(19) Europäisches Patentamt European Patent Office Office européen des brevets

(11) **EP 2 532 306 B1**

(12) **EUROPEAN PATENT SPECIFICATION**

(45) Date of publication and mention of the grant of the patent:
**23.03.2016 Bulletin 2016/12**

(51) Int Cl.:
*A61B 5/18* *(2006.01)*        *A61B 5/0428* *(2006.01)*
*H03F 1/34* *(2006.01)*        *H03G 3/00* *(2006.01)*

(21) Application number: **12171404.2**

(22) Date of filing: **08.06.2012**

(54) **Non-contact sensor for detecting the electrocardiogram of a user**

Kontaktloser Sensor zur Erkennung des Elektrokardiogramms eines Benutzers

Capteur sans contact permettant de détecter l'électrocardiogramme d'un utilisateur

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.06.2011 IT BO20110329**

(43) Date of publication of application:
**12.12.2012 Bulletin 2012/50**

(73) Proprietor: **FERRARI S.p.A.**
**Modena (IT)**

(72) Inventors:
• **Ragnoni, Alessandro**
  **41053 Maranello (IT)**
• **Visconti, Amedeo**
  **10138 Torino (IT)**
• **La Gatta, Antonio**
  **35037 Teolo (IT)**
• **Azzena, Gianfranco**
  **44121 Ferrara (IT)**

(74) Representative: **Bergadano, Mirko et al**
**Studio Torta S.p.A.**
**Via Viotti, 9**
**10121 Torino (IT)**

(56) References cited:
WO-A1-2006/031025    WO-A1-2009/070776
US-A- 3 210 678      US-A- 4 245 649
US-A- 5 650 750      US-A1- 2007 252 656
US-A1- 2008 290 937

## Description

### TECHNICAL FIELD

The present invention relates to a non-contact sensor for detecting the electrocardiogram of a user.

The present invention finds advantageous application in a vehicle for detecting the driver's electrocardiogram, to which the following description will make explicit reference to without, however, any loss of generality.

### PRIOR ART

Recently it has been proposed to detect certain physiological parameters of the driver of a vehicle to assess the driver's psychophysical state and consequently take action (for example, by means of visual and/or audible indicators or even progressively reducing the speed of the vehicle until it stops) when the psychophysical state of the driver clearly worsens. An example of a method for characterizing the psychophysical state of the driver of a vehicle is provided in patent application WO02096694A1.

One of the main physiological parameters that are considered to assess the psychophysical state of the driver is the heart rate (derivable directly from the electrocardiogram).

As described in patent application JP2011024902A2, the use of a contact sensor comprising electrically conductive surfaces that cover part of the steering wheel rim has been proposed to detect the electrocardiogram of the driver of a vehicle; however, this technical solution has various drawbacks as, on one hand, it only works when the driver holds the steering wheel with both hands and, on the other, the electrically conducting surfaces that cover part of the steering wheel rim provide a not very comfortable tactile sensation which, in the long run, causes evident annoyance to the driver.

As described in patent application DE10031822A1, the use of a sensor fixed to the safety belt has been proposed to detect the electrocardiogram of the driver of a vehicle; however, this technical solution is not very comfortable due to the bulk of the sensor and has an aesthetic look that is poorly appreciated both by end users and by car manufacturers. In addition, this technical solution does not work if the driver does not fasten the safety belt and is not applicable to vehicles in which safety belts are not contemplated (for example, trains and buses) or, in any case, where they are not regularly used.

As described in patent application US2007255152A1, the use of a sensor that is incorporated in the seat and comprises a first electrode inserted inside the seat back and a second electrode inserted in the seat squab has been proposed to detect the electrocardiogram of the driver of a vehicle; however, this technical solution has various drawbacks as, on one hand, it requires inserting very large, rigid electrodes that cause a perceivable reduction in comfort and, on the other, have a modest measuring precision, as detection of the electrocardiogram is negatively influenced by undesired and random contact voltages that are picked up by the electrodes and are superimposed on the electrical signals generated by the cardiac muscle during the heartbeat.

WO2009070776A1 discloses a capacitive non-contact biopotential sensor including a sensing plate, an amplifier, and a switching circuit.

US2008290937A1 discloses a constant gain amplifier system with positive and negative feedback based on controlling oscillator loop gain; the amplifier system comprises an oscillator formed by an amplifier and by a constant gain positive feedback network.

US3210678A discloses a feedback stabilized direct coupled amplifier.

US5650750A discloses a common mode signal and circuit fault detection in differential signal detectors for detecting differential mode signals in an environment where differential mode signals co-exist, and might be corrupted by, common mode signals.

US 4245649 A discloses a non-contact sensor according to the preamble of claim 1.

### DESCRIPTION OF THE INVENTION

The object of the present invention is to provide a non-contact sensor for detecting the electrocardiogram of a user, this sensor being devoid of the above-described drawbacks and, in particular, is simple and inexpensive to manufacture.

According to the present invention, a non-contact sensor is provided for detecting the electrocardiogram of a user, in accordance with the appended claims.

### BRIEF DESCRIPTION OF DRAWINGS

The present invention will now be described with reference to the attached drawings, which illustrate some non-limitative examples of embodiment, where:

- Figure 1 is a schematic plan view of a vehicle in which the driver's seat integrates a non-contact sensor that is made according to the present invention and detects the electrocardiogram of the driver;
- Figure 2 is a schematic side view of the driver's seat that integrates the non-contact sensor;
- Figure 3 is a schematic, perspective view of the driver's seat that integrates the non-contact sensor;
- Figure 4 is a circuit diagram of a detecting device of the non-contact sensor;
- Figure 5 is a graph that shows the time trend of an electrical signal provided as output by the detecting device in Figure 4;
- Figure 6 is a circuit diagram of a combiner device of the non-contact sensor;
- Figure 7 is a circuit diagram of a different embodiment of the combiner device of Figure 6; and
- Figure 8 is a circuit diagram of a non-contact sensor without the combiner device of Figures 6 and 7.

## PREFERRED EMBODIMENTS OF THE INVENTION

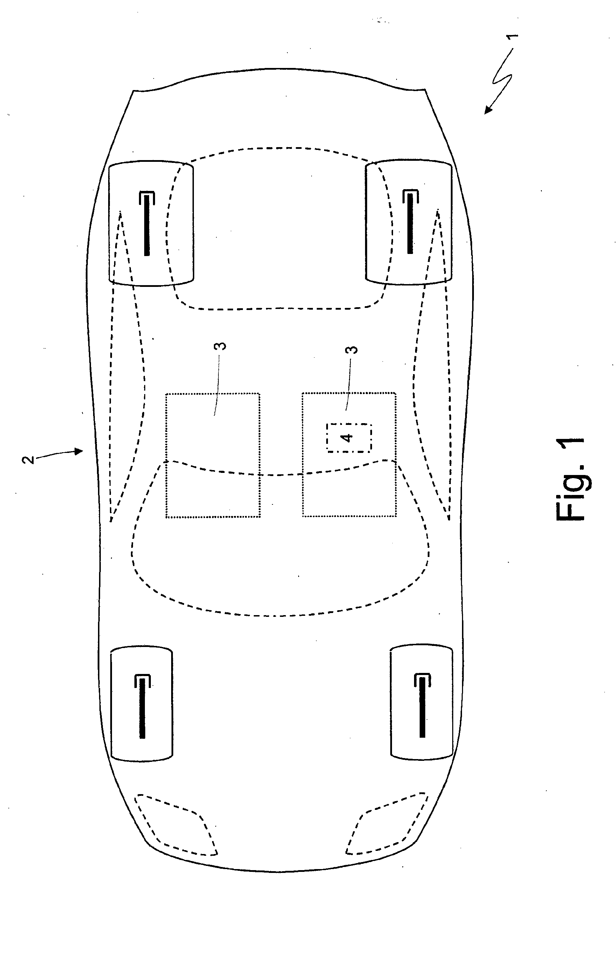

In Figure 1, reference numeral 1 indicates, in its entirety, a vehicle equipped with a two-seat passenger compartment 2 that is fitted with two seats 3 for a driver (left-hand seat) and a possible passenger (right-hand seat).

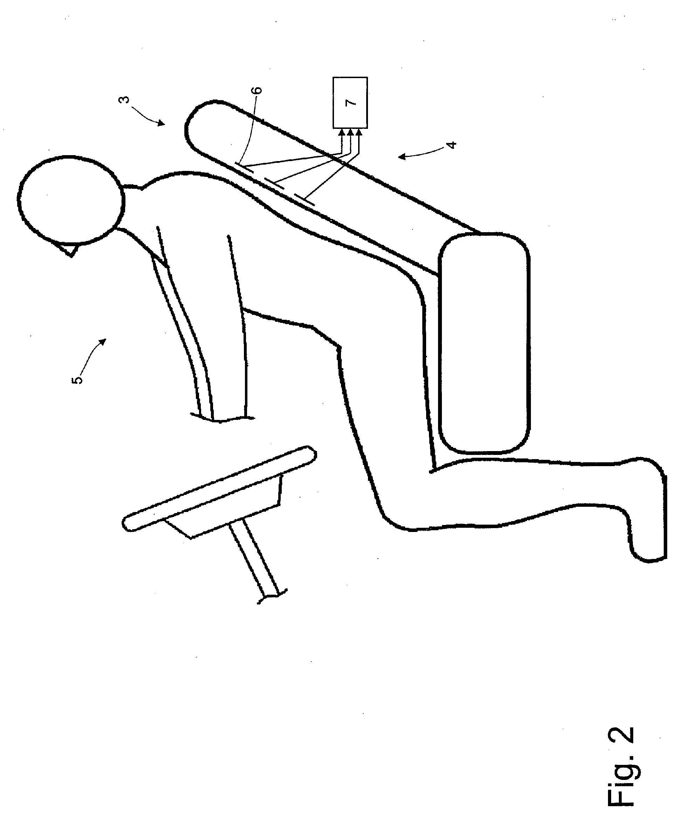

A non-contact sensor 4 is integrated in the driver's seat 3 for detecting the electrocardiogram of a user 5 (in particular of the driver of the vehicle 1, schematically shown in Figure 2).

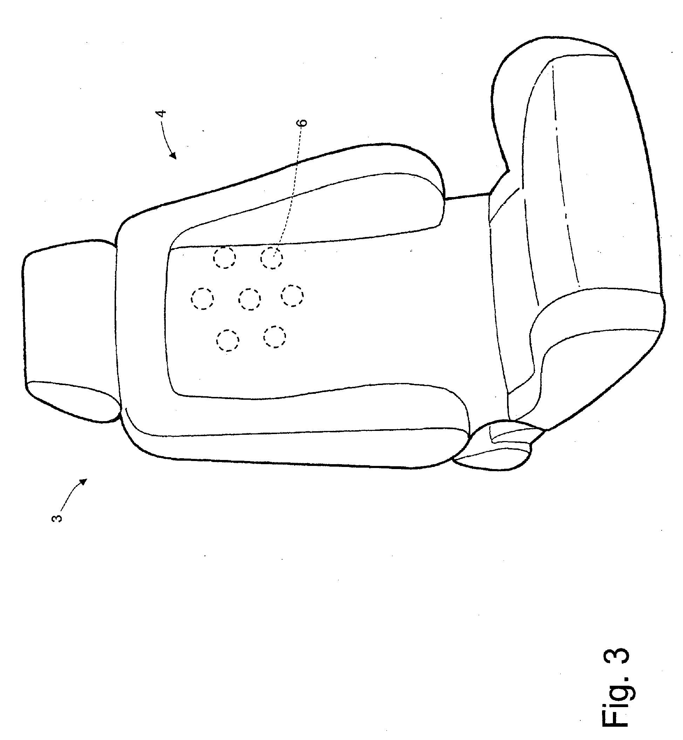

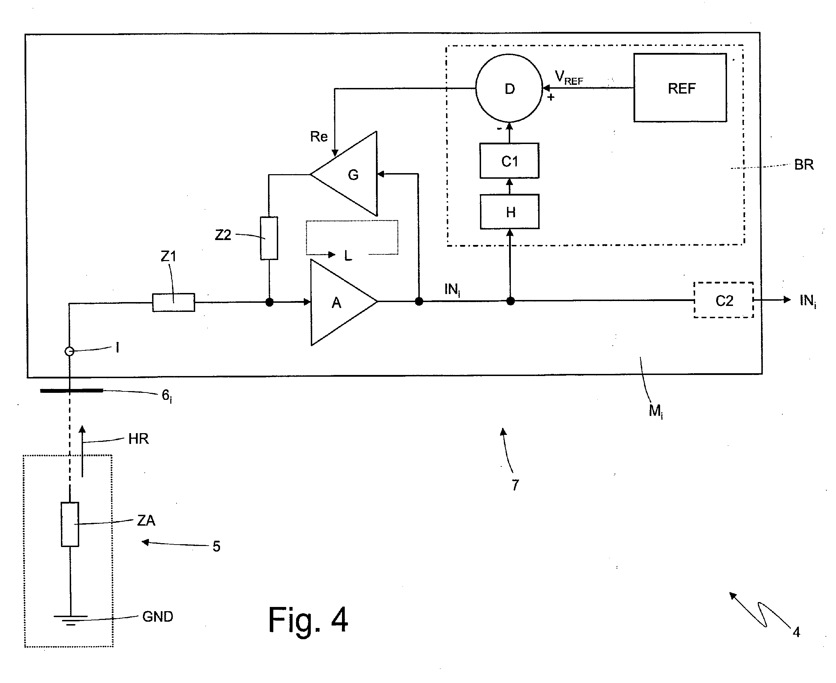

According to that shown in Figures 2 and 3, the sensor 4 comprises a plurality of conducting plates 6 (typically made of a metal material), which are incorporated inside the backrest of the seat 3 in an area corresponding to the position of heart of user 5 and constitute sensor elements that pick up the electrical HR signals (schematically shown in Figure 4) generated by the cardiac muscle during the heartbeat (in particular, the electrical HR signals are constituted by an electric charge fluctuation in the chest of the user 5). In addition, the sensor 4 comprises a processing unit 7 that is electrically connected to the conducting plates 6 and which processes the electrical HR signals picked up by the conducting plates 6 to determine the electrocardiogram of the user 5.

According to a preferred embodiment illustrated in Figure 3, a number of conducting plates 6 are envisaged that are arranged in an area that covers all the positions in which the heart of the user 5 might be found, taking variability in height and build into account; in this way, at least one conducting plate 6 will always be found in the optimal position (i.e. that of maximum efficiency) to pick up the electrical HR signals generated by the cardiac muscle during the heartbeat. Typically, two to seven conducting plates 6 are provided, which are normally arranged according to an evenly distributed layout, for example, hexagonal (as shown in Figure 3) or square. By way of example, the set of conducting plates 6 occupies an overall area of approximately 300 mm x 200 mm, while a single conducting plate 6 has an area of approximately 1600 $mm^2$ (i.e., if it is round, it has a diameter of approximately 20-25 mm).

In the embodiment illustrated in Figures 4, 6 and 7, for each conducting plate $6_i$ (or rather, for the i'th conducting plate), the processing unit 7 comprises a corresponding detecting device $M_i$, which is electrically connected to the conducting plate $6_i$ and has the function of detecting and amplifying the electrical HR signals that are generated by the cardiac muscle during the heartbeat and are picked up by the conducting plate $6_i$.

According to that shown in Figure 4, each detecting device $M_i$ is connected to a corresponding conducting plate $6_i$ that receives the electrical HR signals from the user 5 and which can be electrically represented with an impedance ZA to ground (GND). The two impedances Z1 and Z2 constitute a voltage divider and therefore the voltage at the input of an amplifier block A is equal to the sum of the voltage coming from the input I and the voltage coming from the output of an attenuator block G that is arranged in parallel and in opposition to the amplifier block A (i.e. the output of the amplifier block A is connected to the input of the attenuator block G and the input of the amplifier block A is connected to the output of the attenuator block G).

The amplifier block A is a very-low-noise instrumentation amplifier, causes signal amplification (i.e. the output voltage Vu is higher than the input voltage Vi) and has a transfer function (TF) represented by the equation Vu/Vi=ka, where the gain "ka" is fixed (i.e. always constant) and is greater than 1 and normally in the range between 1 and 100. The attenuator block G causes signal attenuation (i.e. the output voltage Vu is lower than the input voltage Vi) and has a transfer function (TF) represented by the equation Vu/Vi=kg*Re, where the attenuation "kg*Re" as a whole is less than 1 and is variable; in particular, "kg" is a constant less than 1, while "Re" is a control signal that determines a variation in the attenuation "kg*Re" of the attenuator block G. In other words, the attenuator block G has an attenuation that is less than 1 and is variable as a function of the control signal Re.

When the mesh L constituted by the amplifier block A and the attenuator block G has an overall unitary gain (i.e. close to unity) it will tend to self-oscillate, or rather trigger periodic oscillations within itself (the frequency of which also depends of the circuit's stray capacitance). On one hand, the self-oscillation of the mesh L is negative as it introduces an oscillating signal into the main signal received from the conducting plate $6_i$ that constitutes noise; however, on the other hand, the self-oscillation of the mesh L is positive as it gives the mesh L an extremely high input impedance (mathematically, in this condition, the input impedance of the mesh L diverges to infinity and, in practice, the input impedance of the mesh L is in the order of $10^{18}$ Ohm in this condition). The quantities of the detecting device $M_i$ are chosen to ensure that the self-oscillation frequency of the mesh L is significantly higher (at the very least twice, but typically at least 4-6 times more) than the maximum heart rate; in other words, the quantities of the detecting device $M_i$ are chosen such that the self-oscillation frequency of the mesh L is above 400-600 Hz (if necessary, capacitors experimentally sized to increase the self-oscillation frequency of the mesh L can be added to the detecting device $M_i$).

Self-oscillation occurs when the following equation is satisfied:

$$\frac{ka \cdot kg \cdot \mathrm{Re} \cdot (Z1 + Za)}{Z2 + Z1 + Za} = 1$$

where Za is the coupling impedance to the environment of the conducting plate $6_i$; obviously, the impedance Za normally has a very high value (more than $10^6$ Ohm), but unfortunately is unknown and variable with time. Therefore, the self-oscillation condition is not known beforehand and is not even steady, but changes as the surrounding conditions change (position of the user 5 with respect to the conducting plate $6_i$, ambient humidity, proximity of other objects...). Thus, in order to always keep the mesh L in self-oscillation conditions, the control signal Re is constantly updated (or rather kept at the value that determines the self-oscillation of the mesh L).

In particular, the control signal Re is generated by a control block BR, which considers the high-frequency oscillating content (that is, at frequencies much higher than the heart rate, for example, above 500 Hz) of the $IN_i$ output signal from the amplifier block A, as the high-frequency oscillating content of the $IN_i$ output signal is basically formed by the oscillations caused by the self-oscillation of the mesh L. The BR block feedback controls the control signal Re so that the high-frequency oscillating content of the output signal $IN_i$ is constant and equal to a desired value represented by a reference voltage $V_{REF}$. The reference voltage $V_{REF}$ is chosen such that the high-frequency oscillating content of the $IN_i$ output signal (i.e. the self-oscillation of the mesh L) is approximately 5-10% of the overall $IN_i$ output signal; in this way, the self-oscillation of the mesh L does not have a significant negative effect on the acquisition of the electrical HR signals.

The control block BR comprises a rectifier block H (in particular a dual half-wave rectifier) that receives the $IN_i$ output signal of the amplifier block A as input and supplies the rectified (i.e. always positive) $IN_i$ output signal. In addition, the control block BR comprises a high-pass filter C1 (which cuts out the "low" frequencies, i.e. those less than the self-oscillation frequency of the mesh L and lets through the "high" frequencies, i.e. those of the order of the self-oscillation frequency of the mesh L), which filters the output signal of the rectifier block H to determine the high-frequency oscillating content of the $IN_i$ output signal of the amplifier block A. The high-frequency oscillating content of the $IN_i$ output signal of the amplifier block A determined by the high-pass filter C1 is compared in an subtractor block D with the reference voltage $V_{REF}$ provided by a reference block REF and the output of the subtractor block D (i.e. the deviation between the desired value and the effective value of the high-frequency oscillating content of the $IN_i$ output signal) constitutes the control signal Re. Obviously, the subtractor block D could integrate a controller that further processes the difference between the reference voltage $V_{REF}$ and the high-frequency oscillating content determined by the high-pass filter C1 to calculate the control signal Re of the attenuator block G.

In other words, the control signal Re of the attenuator block G is feedback controlled to keep the high-frequency oscillating content of the $IN_i$ output signal of the amplifier block A equal to a predefined desired value. As also mentioned in the foregoing, the reference voltage $V_{REF}$ is chosen experimentally to bring the level of self-oscillation of the mesh L to have an amplitude at least ten times lower than the overall $IN_i$ output signal.

The main characteristic of the detecting device $M_i$ is the extremely high input impedance (i.e. the extremely high impedance seen from the conducting plate $6_i$), which is of the order of $10^{18}$ Ohm (practically infinite) and is achieved by always keeping the mesh L in self-oscillation conditions. Thanks to the extremely high input impedance of the detecting device $M_i$, the detecting device $M_i$ "senses" the electrical HR signals generated by the cardiac muscle during the heartbeat without any significant passage of electric current (in other words, the conducting plate $6_i$ acts like the plate of a capacitor that "senses" the fluctuations in electrical charge without any significant passage of electric current). Thanks to the substantial absence of electric current flow, the detecting device $M_i$ is completely immune to the negative effect of random contact voltages that might occur on the skin of the user 5 and could be picked up by the conducting plate $6_i$.

According to a possible embodiment illustrated with a hatched line, the detecting device $M_i$ could also include a further low-pass filter C2 (which cuts out the "high" frequencies, i.e. those of the order of the self-oscillation frequency of the mesh L, and lets through the "low" frequencies, i.e. those of the order of the heart rate), which filters the output signal $IN_i$ of the amplifier block A before supplying the output signal $IN_i$ as output from the detecting device $M_i$. The function of the low-pass filter C2 is to "clean" the component of self-oscillation of the mesh L from the output signal $IN_i$. Obviously, the low-pass filter C2 is arranged downstream of the control block BR, as the operation of the control block BR is based on the presence of the component of self-oscillation of the mesh L in the output signal $IN_i$.

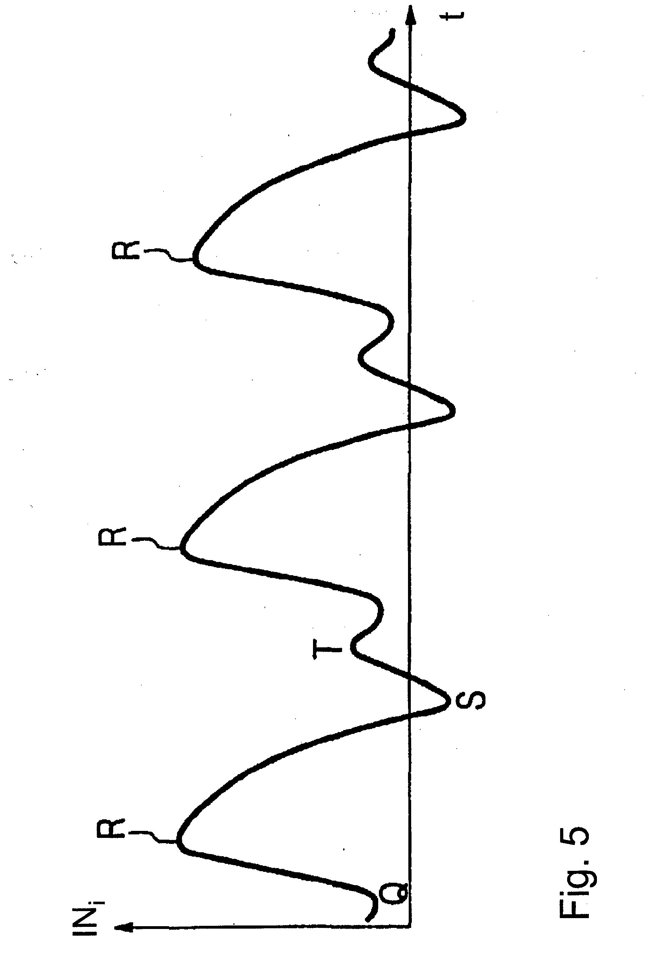

Figure 5 shown a sample output signal $IN_i$ (filtered by the low-pass filter C2) that is supplied at the output of the detecting device $M_i$ (obviously when the corresponding conducting plate $6_i$ happens to be in an optimal position). It should be noted how the output signal $IN_i$ imitates the classical waveform of an electrocardiogram. The main part of an electrocardiogram wave is the QRS, which represents the ventricular depolarization; in particular, a sudden downward wave (Q) can be observed in the electrocardiogram trace, which constitutes the beginning of the QRS complex, followed by a positive wave (R - the top point of the electrocardiogram used to calculate the heart rate) and the negative waves that are preceded by positive waves are called S. There is a wave T after the QRS that represents the ventricular repolarization, during which the cells of the cardiac muscle return to a rest state, permitting a successive stimulation.

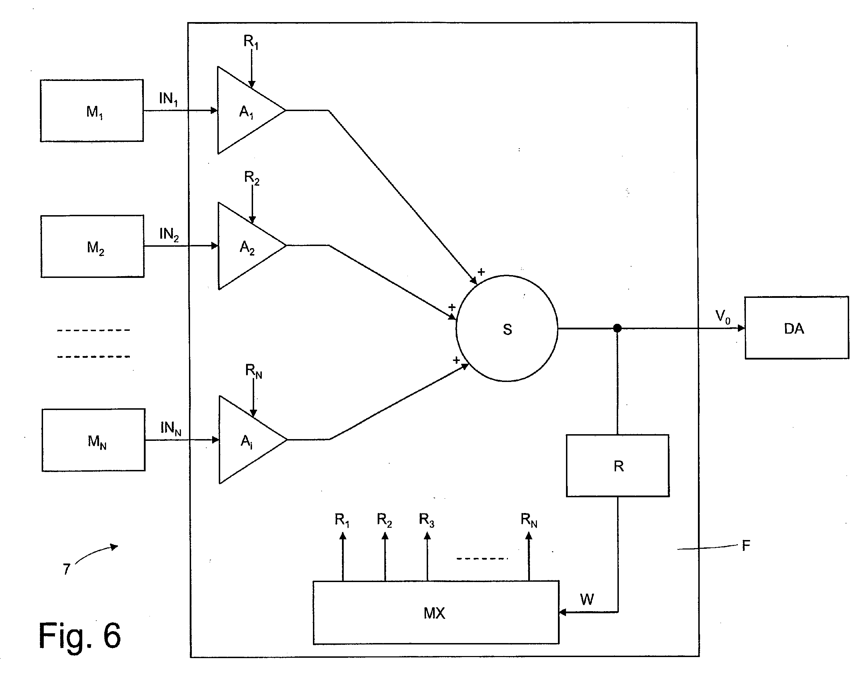

According to that shown in Figure 6, the processing unit 7 of the sensor 4 comprises a combiner device F that receives in input all of the output signals $IN_i$ supplied by the detecting devices $M_i$ and combines them, according to a method described further on, to determine a combined signal $V_0$ that represents the best possible signal to determine the electrocardiogram of the user 5. The combined signal $V_0$ is supplied to an analysis device DA that analyses the combined signal $V_0$ in a known manner to determine the desired cardiac parameters (for example, the heart rate).

According to a preferred embodiment, the combined signal $V_0$ is a linear combination of the output signals $IN_i$ supplied by the detecting devices $M_i$ that envisages the use of weights $R_i$ that assume three values: +1,0,-1. In other words, the combined signal $V_0$ is provide by the following equation:

$$V_0 = \Sigma\ IN_i\ \cdot\ R_i = IN_1\ \cdot\ R_1 + IN_2\ \cdot\ R_2 + \ldots + IN_N\ \cdot\ R_N$$

where the weights $R_i$ can assume three values: +1,0,-1.
In other words, each block $A_i$ has the following transfer functions:

$$R_i=1 \qquad \rightarrow \qquad Vu/Vi=1$$

$$R_i=0 \qquad \rightarrow \qquad Vu/Vi=0$$

$$R_i=-1 \qquad \rightarrow \qquad Vu/Vi=-1$$

The values $R_1...R_N$ are periodically determined by the permutation block MX, as described below.

The combined signal $V_0$ is processed by a QRS recognition block R (as previously described, the "QRS" is the main part of an electrocardiogram wave) that returns a numeric value W proportional to the "fitting" of the combined signal $V_0$ with a QRS wave; in other words, the value W is a "score" that increases the more the combined signal $V_0$ "resembles" a QRS wave (i.e. has the same shape). The QRS recognition block R is already known in the literature and is normally implemented with the measurement of the absolute maximum of cross-correlation between the combined signal $V_0$ and a sample QRS signal.

The permutation block MX periodically tests all the possible combinations of the weights $R_i$ (namely $3^N$ combinations where N is the number of detecting devices $M_i$, or rather conducting plates $6_i$) to find the particular combination of weights $R_i$ that enable obtaining the "best" combined signal $V_0$ (or rather the combined signal $V_0$ that has the highest W value, i.e. the closest fit with a QRS wave). In other words, by varying the weights $R_i$, a maximum value search is performed in which the quantity to maximize is the W value, i.e. a search for the combination of weights $R_i$ that enables the W value to be maximized. It is important to observe that the values of the weights $R_i$ can also be negative, because a better result is often achieved by combining the output signals $IN_i$ originating from two detecting devices $M_i$ (namely from two conducting plates 6) in phase opposition.

In use, the permutation block MX carries out the search for the best combination of weights $R_i$ with a predetermined frequency (for example, every 1-3 minutes): at the end of the search for the best combination of weights $R_i$, the combination of weights $R_i$ found to be the best is "frozen" and used until the next search operation. Obviously, the combined signal $V_0$ cannot be used (or rather it cannot be supplied to the analysis device DA) during the search for the best combination of weights $R_i$ as there are continuous alterations due to the continual changes in the weights $R_i$ while searching for the best combination of weights $R_i$.

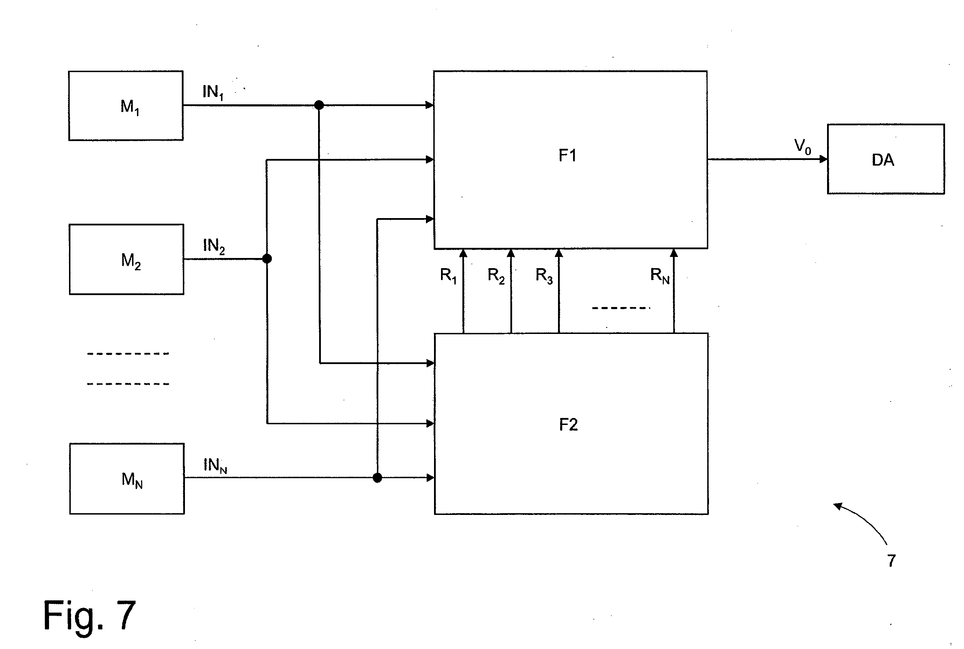

In the embodiment illustrated in Figure 7, two combiner devices F1 and F2 are used, these being arranged in parallel to each other. The main combiner device F1 does not have (or does not use) the permutation block MX (i.e. it does not make any changes the combination of weights $R_i$) and uses the combination of weights $R_i$ provided by combiner device F2 to determine the combined signal $V_0$ that is supplied to the analysis device DA. Instead, the secondary combiner device F2 does not supply the combined signal $V_0$ to the analysis device DA and has the sole function of determining the best combination of weights $R_i$, which is then supplied to the main combiner device F1 that uses the best combination of weights $R_i$ to determine the combined signal $V_0$ that is supplied to the analysis device DA. In other words, the tasks of the two combiner devices F1 and F2 are divided: the main combiner device F1 has the sole function of calculating combined signal $V_0$ that is supplied to the analysis device DA, while the secondary combiner device F2 has the sole function of determining the best combination of weights $R_i$. In this way, there is never any interruption in the combined signal $V_0$ that is supplied to the analysis device DA.

According to a preferred embodiment, the best combination of weights $R_i$ is only changed if it guarantees a significant improvement (i.e. only if the increase in the W value is significant) and only if a sufficient time interval has passed since the previous change (the duration of this time interval can decrease as the improvement guaranteed by the change increases). In this way, changing the best combination of weights $R_i$ too frequently is avoided, ensuring greater continuity (i.e. avoiding too many discontinuities) in the analysis performed by the analysis device DA.

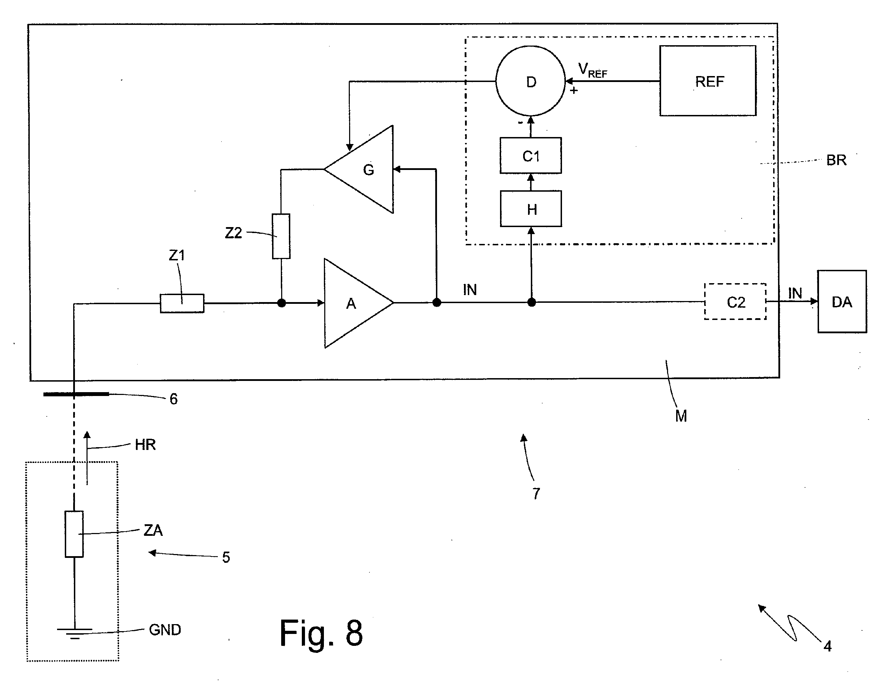

In accordance with the embodiment illustrated in Figure 8, the sensor 4 comprises a single conducting plate 6 and therefore comprises a single detecting device M; in this case, the combiner device F is obviously absent and the signal IN originating from the detecting device M is supplied directly to the analysis device DA. This embodiment can be used when there is the certainty that the sole conducting plate 6 is always arranged in proximity to the heart of the user 5.

In the above-described embodiment, the sensor 4 is (or at least the conducting plates 6 of the sensor 4 are) integrated in the backrest of the driver's seat 3 of a vehicle 1. It is evident that the sensor 4 can also be used in other environments: for example, a conducting plate 6 of the sensor 4 could be integrated in a garment worn by the user 5 or could be part of a portable object that is manually brought close to the heart of the user 5.

The above-described non-contact sensor 4 has numerous advantages.

First of all, the above-described sensor 4 is simple and inexpensive to manufacture, as it envisages the use of low-cost components that are readily available on the market.

Furthermore, the above-described sensor 4 is extremely light and of easy and immediate integration in a seat of a vehicle. In particular, the conducting plates 6 are small in size and therefore their presence inside the backrest of the seat 3 is not perceived by the user 5 and does not cause any deterioration in comfort for the user 5.

Finally, but not of lesser importance, the above-described sensor 4 is capable of detecting the electrocardiogram of the user 5 in a very accurate manner even when the conducting plate 6 (or the conducting plates 6) is (are) at a certain distance from the skin of the of the user 5 (even up to 1-3 cm from the skin of the user, and consequently detecting with ease even through the clothes worn by the user 5).

**Claims**

1.  A non-contact sensor (4) for detecting the electrocardiogram of a user (5); the sensor (4) comprises:

    at least one conducting plate (6) suitable for being arranged in proximity to the heart of the user (5); and
    a detecting device (M), which is electrically connected to the conducting plate (6) and has the function of detecting and
    amplifying the electrical signals (HR) that are generated by the cardiac muscle during the heartbeat and are picked up by the conducting plate (6);

    wherein the detecting device (M) comprises:

    a mesh (L), which is electrically connected to the conducting plate (6) and is able to self-oscillate; and
    a control block (BR), which controls the mesh (L) so as to always keep the mesh (L) in a condition of self-oscillation;

    the sensor (4) is **characterized in that** the detecting device (M) comprises:

    an amplifier block (A), which is part of the mesh (L), has a constant gain (ka), has its input connected to the conducting plate (6) and provides as output the output signal (IN) of the detecting device (M); and
    an attenuator block (G), which has variable attenuation (kg*Re) and is arranged in parallel and in opposition to the amplifier block (A) so as to form, together with the amplifier block (A), the mesh (L);
    wherein the control block (BR) adjusts the attenuation (kg*Re) of the attenuator block (G) so as to always keep the mesh (L) in a condition of self-oscillation by performing a feedback control that keeps the high-frequency oscillating content in the output signal (IN) of the amplifier block (A) equal to a desired value.

2.  The sensor (4) according to claim 1, wherein the self-oscillation of the mesh (L) has a frequency that is significantly higher than the maximum heart rate.

3. The sensor (4) according to claim 1 or 2, wherein the self-oscillation of the mesh has an amplitude that is a fraction, in particular not more than 5-10%, of the amplitude of the output signal (IN) of the detecting device (M).

4. The sensor (4) according to claim 1, 2 or 3, wherein the control block (BR) comprises:

   a rectifier block (H), which receives the output signal (IN) of the amplifier block (A) as input and provides the rectified output signal as output;
   a high-pass filter (C1), which cuts out the frequencies lower than the self-oscillation frequency of the mesh (L) and filters the output signal of the rectifier block (H) to determine the high-frequency oscillating content of the output signal (IN) of the amplifier block (A);
   a reference block (REF), which generates a reference voltage ($V_{REF}$) corresponding to the desired value of the high-frequency oscillating content; and
   a subtractor block (D), which generates a control signal (Re) for the attenuation (kg*Re) of the attenuator block (G) by comparing the reference voltage ($V_{REF}$) with the high-frequency oscillating content determined by the high-pass filter (C1).

5. The sensor (4) according to any of claims 1 to 4, wherein the detecting device (M) comprises a low-pass filter (C2), which cuts out the frequencies higher than the maximum heart rate and is arranged downstream of the mesh (L) and the control block (BR).

6. The sensor (4) according to any of claims 1 to 5 and comprising an analysis device (DA), which is directly connected to the output of the detecting device (M) and analyses the output signal (IN) of the detecting device (M) to determine desired cardiac parameters.

7. The sensor (4) according to any of claims 1 to 5 and comprising:

   a plurality of conducting plates ($6_i$);
   a plurality of detecting devices ($M_i$), each of which is electrically connected to a corresponding conducting plate ($6_i$); and
   a combiner device (F; F1) that receives all the output signals ($IN_i$) provided by the detecting devices ($M_i$) as input and combines them together to determine a combined signal ($V_0$), which represents the best possible signal for determining the electrocardiogram of the user (5).

8. The sensor (4) according to claim 7 and comprising an analysis device (DA), which is directly connected to the output of the combiner device (F; F1, F2) and analyses the combined signal ($V_0$) to determine desired cardiac parameters.

9. The sensor (4) according to claim 7 or 8, wherein the combined signal ($V_0$) is a linear combination of the output signals ($IN_i$) provided by the detecting devices ($M_i$) resulting from the following equation:

$$V_0 = \Sigma\ IN_i \cdot R_i = IN_1 \cdot R_1 + IN_2 \cdot R_2 + ... + IN_n \cdot R_n$$

   $V_0$ combined signal;
   $IN_i$ output signals provided by the detecting devices ($M_i$);
   $R_i$ weights, which can assume three values: +1,0,-1.

10. The sensor (4) according to claim 9, wherein the combiner device (F; F1, F2) comprises a permutation block (MX), which cyclically tests all the possible combinations of the weights ($R_i$) to find the optimal combination of the weights ($R_i$) that enables achieving the best combined signal ($V_0$).

11. The sensor (4) according to claim 10, wherein the combiner device (F; F1, F2) comprises a QRS recognition block (R), which analyses the combined signal ($V_0$) and returns a numeric value (W) proportional to the fitting of the combined signal ($V_0$) with a QRS wave; the best combined signal ($V_0$) is the combined signal ($V_0$) that has the

highest numeric value (W) returned by the QRS recognition block (R).

12. The sensor (4) according to claim 10 or 11 and comprising:

a first combiner device (F2), which determines the optimal combination of the weights ($R_i$) that enables achieving the best combined signal ($V_0$); and
a second combiner device (F1), which uses the optimal combination of the weights ($R_i$) determined by the first combiner device (F2) to determine the combined signal ($V_0$).

13. A vehicle (1) comprising a passenger compartment (2) equipped with at least one seat (3) in which a non-contact sensor (4) according to any of claims 1 to 12 is integrated for detecting the electrocardiogram of a user (5).


**Patentansprüche**

1. Kontaktloser Sensor (4) zum Detektieren des Elektrokardiogramms eines Benutzers (5); wobei der Sensor (4) aufweist:

mindestens eine leitende Platte (6), die zum Anordnen in der Nähe des Herzens des Benutzers (5) geeignet ist; und
eine Detektionsvorrichtung (M), die mit der leitenden Platte (6) elektrisch verbunden ist und die Funktion zum Detektieren und Verstärken der elektrischen Signale (HR) hat, die durch den Herzmuskel während des Herzschlags erzeugt und durch die leitende Platte (6) aufgenommen werden;
wobei die Detektionsvorrichtung (M) aufweist:

eine Masche (L), die mit der leitenden Platte (6) elektrisch verbunden ist und selbstschwingen kann; und
einen Steuerblock (BR), der die Masche (L) so steuert, dass er die Masche (L) stets in einem Selbstschwingungszustand hält;
wobei der Sensor (4) **dadurch gekennzeichnet ist, dass** die Detektionsvorrichtung (M) aufweist:

einen Verstärkerblock (A), der Teil der Masche (L) ist, eine konstante Verstärkung (ka) hat, seinen Eingang mit der leitenden Platte (6) verbunden hat und als Ausgabe das Ausgangssignal (IN) der Detektionsvorrichtung (M) bereitstellt; und
einen Dämpferblock (G), der eine variable Dämpfung (kg*Re) hat sowie parallel und in Gegenüberstellung zum Verstärkerblock (A) angeordnet ist, um zusammen mit dem Verstärkerblock (A) die Masche (L) zu bilden;
wobei der Steuerblock (BR) die Dämpfung (kg*Re) des Dämpferblocks (G) so einstellt, dass die Masche (L) stets in einem Selbstschwingungszustand gehalten wird, indem eine Rückführregelung durchgeführt wird, die den hochfrequenten Schwingungsgehalt im Ausgangssignal (IN) des Verstärkerblocks (A) gleich einem gewünschten Wert hält.

2. Sensor (4) nach Anspruch 1, wobei die Selbstschwingung der Masche (L) eine Frequenz hat, die erheblich höher als die maximale Herzfrequenz ist.

3. Sensor (4) nach Anspruch 1 oder 2, wobei die Selbstschwingung der Masche eine Amplitude hat, die einen Bruchteil, insbesondere höchstens 5-10 %, der Amplitude des Ausgangssignals (IN) der Detektionsvorrichtung (M) beträgt.

4. Sensor (4) nach Anspruch 1, 2 oder 3, wobei der Steuerblock (BR) aufweist:

einen Gleichrichterblock (H), der das Ausgangssignal (IN) des Verstärkerblocks (A) als Eingabe empfängt und das gleichgerichtete Ausgangssignal als Ausgabe bereitstellt;
ein Hochpassfilter (C1), das die Frequenzen unterhalb der Selbstschwingungsfrequenz der Masche (L) ausschneidet und das Ausgangssignal des Gleichrichterblocks (H) filtert, um den hochfrequenten Schwingungsgehalt des Ausgangssignals (IN) des Verstärkerblocks (A) zu bestimmen;
einen Referenzblock (REF), der eine Referenzspannung ($V_{REF}$) in Entsprechung zum gewünschten Wert des hochfrequenten Schwingungsgehalts erzeugt; und
einen Subtrahiererblock (D), der ein Steuersignal (Re) für die Dämpfung (kg*Re) des Dämpferblocks (G) erzeugt, indem er die Referenzspannung ($V_{REF}$) mit dem hochfrequenten Schwingungsgehalt vergleicht, der durch das Hochpassfilter (C1) bestimmt wird.

5. Sensor (4) nach einem der Ansprüche 1 bis 4, wobei die Detektionsvorrichtung (M) ein Tiefpassfilter (C2) aufweist, das die Frequenzen oberhalb der maximalen Herzfrequenz ausschneidet sowie der Masche (L) und dem Steuerblock (BR) nachgelagert ist.

6. Sensor (4) nach einem der Ansprüche 1 bis 5 und mit einer Analysenvorrichtung (DA), die mit dem Ausgang der Detektionsvorrichtung (M) direkt verbunden ist und das Ausgangssignal (IN) der Detektionsvorrichtung (M) analysiert, um gewünschte Herzparameter zu bestimmen.

7. Sensor (4) nach einem der Ansprüche 1 bis 5 und mit:

   mehreren leitenden Platten ($6_i$);
   mehreren Detektionsvorrichtungen ($M_i$), von denen jede mit einer entsprechenden leitenden Platte ($6_i$) elektrisch verbunden ist; und
   einer Kombinierervorrichtung (F; F1), die alle durch die Detektionsvorrichtungen ($M_i$) bereitgestellten Ausgangssignale ($IN_i$) als Eingabe empfängt und sie miteinander kombiniert, um ein kombiniertes Signal ($V_0$) zu bestimmen, das das bestmögliche Signal zum Bestimmen des Elektrokardiogramms des Benutzers (5) darstellt.

8. Sensor (4) nach Anspruch 7 und mit einer Analysenvorrichtung (DA), die mit dem Ausgang der Kombinierervorrichtung (F; F1, F2) direkt verbunden ist und das kombinierte Signal ($V_0$) analysiert, um gewünschte Herzparameter zu bestimmen.

9. Sensor (4) nach Anspruch 7 oder 8, wobei das kombinierte Signal ($V_0$) eine lineare Kombination der durch die Detektionsvorrichtungen ($M_i$) bereitgestellten Ausgangssignale ($IN_i$) ist, die sich aus der folgenden Gleichung ergibt:

$$V_0 = \Sigma\ IN_i \cdot R_i = IN_1 \cdot R_1 + IN_2 \cdot R_2 + \ldots + IN_n \cdot R_n,$$

   wobei:

   $V_0$: kombiniertes Signal;
   $IN_i$: durch die Detektionsvorrichtungen ($M_i$) bereitgestellte Ausgangssignale;
   $R_i$: Gewichte, die drei Werte +1, 0, -1 annehmen können.

10. Sensor (4) nach Anspruch 9, wobei die Kombinierervorrichtung (F; F1, F2) einen Permutationsblock (MX) aufweist, der alle möglichen Kombinationen der Gewichte ($R_i$) zyklisch testet, um die optimale Kombination der Gewichte ($R_i$) zu ermitteln, die ermöglicht, das beste kombinierte Signal ($V_0$) zu erreichen.

11. Sensor (4) nach Anspruch 10, wobei die Kombinierervorrichtung (F; F1, F2) einen QRS-Erkennungsblock (R) aufweist, der das kombinierte Signal ($V_0$) analysiert und einen Zahlenwert (W) zurückreicht, der proportional zur Anpassung des kombinierten Signals ($V_0$) an eine QRS-Welle ist; wobei das beste kombinierte Signal ($V_0$) das kombinierte Signal ($V_0$) ist, das den höchsten Zahlenwert (W) hat, der durch den QRS-Erkennungsblock zurückgereicht wird.

12. Sensor (4) nach Anspruch 10 oder 11 und mit:

   einer ersten Kombinierervorrichtung (F2), die die optimale Kombination der Gewichte ($R_i$) bestimmt, die ermöglicht, das beste kombinierte Signal ($V_0$) zu erreichen; und
   einer zweiten Kombinierervorrichtung (F1), die die durch die erste Kombinierervorrichtung (F2) bestimmte optimale Kombination der Gewichte ($R_i$) verwendet, um das kombinierte Signal ($V_0$) zu bestimmen.

13. Fahrzeug (1) mit einem Fahrgastraum (2), der mit mindestens einem Sitz (3) ausgestattet ist, in dem ein kontaktloser Sensor (4) nach einem der Ansprüche 1 bis 12 zum Detektieren des Elektrokardiogramms eines Benutzers (5) integriert ist.

**Revendications**

1. Capteur sans contact (4) pour détecter l'électrocardiogramme d'un utilisateur (5) ; le capteur (4) comprenant :

 au moins une plaque conductrice (6) susceptible d'être disposée à proximité du coeur de l'utilisateur (5) ; et
 un dispositif de détection (M), qui est électriquement connecté à la plaque conductrice (6), et qui a pour fonction de détecter et d'amplifier les signaux électriques (HR) qui sont générés par le muscle cardiaque durant le battement cardiaque et qui sont saisis par la plaque conductrice (6) ;

 dans lequel le dispositif de détection (M) comprend :

 une maille (L), qui est électriquement connectée à la plaque conductrice (6), et qui est apte à auto-osciller ; et
 un bloc de commande (BR), qui commande la maille (L) de façon à toujours maintenir la maille (L) dans une condition d'auto-oscillation ;
 le capteur (4) étant **caractérisé en ce que** le dispositif de détection (M) comprend :

 un bloc amplificateur (A), qui fait partie de la maille (L), qui a un gain constant (ka), qui a son entrée connectée à la plaque conductrice (6), et qui délivre en sortie le signal de sortie (IN) du dispositif de détection (M) ; et
 un bloc atténuateur (G), qui a une atténuation variable (kg*Re), et qui est disposé en parallèle et en opposition au bloc amplificateur (A) de façon à former, avec le bloc amplificateur (A), la maille (L) ;
 dans lequel le bloc de commande (BR) ajuste l'atténuation (kg*Re) du bloc atténuateur (G) de façon à toujours maintenir la maille (L) dans une condition d'auto-oscillation par la réalisation d'une commande de rétroaction qui maintient le contenu oscillant à haute fréquence dans le signal de sortie (IN) du bloc amplificateur (A) égal à une valeur désirée.

2. Capteur (4) selon la revendication 1, dans lequel l'auto-oscillation de la maille (L) a une fréquence qui est significativement supérieure au rythme cardiaque maximal.

3. Capteur (4) selon la revendication 1 ou 2, dans lequel l'auto-oscillation de la maille a une amplitude qui est une fraction, en particulier pas supérieure à 5 à 10 %, de l'amplitude du signal de sortie (IN) du dispositif de détection (M).

4. Capteur (4) selon la revendication 1, 2 ou 3, dans lequel le bloc de commande (BR) comprend :

 un bloc redresseur (H), qui reçoit le signal de sortie (IN) du bloc amplificateur (A) en entrée et qui délivre le signal de sortie redressé en sortie ;
 un filtre passe-haut (C1), qui coupe les fréquences inférieures à la fréquence d'auto-oscillation de la maille (L), et qui filtre le signal de sortie du bloc redresseur (H) de façon à déterminer le contenu oscillant à haute fréquence du signal de sortie (IN) du bloc amplificateur (A) ;
 un bloc de référence (REF), qui génère une tension de référence ($V_{REF}$) correspondant à la valeur désirée du contenu oscillant à haute fréquence ; et
 un bloc soustracteur (D), qui génère un signal de commande (Re) pour l'atténuation (kg*Re) du bloc atténuateur (G) par la comparaison de la tension de référence ($V_{REF}$) au contenu oscillant à haute fréquence déterminé par le filtre passe-haut (C1).

5. Capteur (4) selon l'une quelconque des revendications 1 à 4, dans lequel le dispositif de détection (M) comprend un filtre passe-bas (C2), qui coupe les fréquences supérieures au rythme cardiaque maximal, et qui est disposé en aval de la maille (L) du bloc de commande (BR).

6. Capteur (4) selon l'une quelconque des revendications 1 à 5, et comprenant un dispositif d'analyse (DA), qui est directement connecté à la sortie du dispositif de détection (M), et qui analyse le signal de sortie (IN) du dispositif de détection (M) de façon à déterminer des paramètres cardiaques désirés.

7. Capteur (4) selon l'une quelconque des revendications 1 à 5, et comprenant :

 une pluralité de plaques conductrices ($6_i$) ;
 une pluralité de dispositifs de détection ($M_i$), dont chacun est électriquement connecté à une plaque conductrice correspondante ($6_i$) ; et

un dispositif de mélangeur (F ; F1), qui reçoit tous les signaux de sortie ($IN_i$) délivrés par les dispositifs de détection ($M_i$) en entrée, et qui les combine les uns aux autres de façon à déterminer un signal combiné ($V_0$), qui représente le meilleur signal possible pour déterminer l'électrocardiogramme de l'utilisateur (5).

8.  Capteur (4) selon la revendication 7, et comprenant un dispositif d'analyse (DA), qui est directement connecté à la sortie du dispositif de mélangeur (F ; F1, F2), et qui analyse le signal combiné ($V_0$) de façon à déterminer des paramètres cardiaques désirés.

9.  Capteur (4) selon la revendication 7 ou 8, dans lequel le signal combiné ($V_0$) est une combinaison linéaire des signaux de sortie ($IN_i$) délivrés par les dispositifs de détection ($M_i$), résultant de l'équation suivante :

$$V_0 = \Sigma\ IN_i \cdot R_i = IN_1 \cdot R_1 + IN_2 \cdot R_2 + ... + IN_n \cdot R_n$$

$V_0$ : signal combiné ;

$IN_i$ : signaux de sortie délivrés par les dispositifs de détection ($M_i$) ;

$R_i$ : poids, qui peuvent prendre trois valeurs : +1, 0, -1.

10.  Capteur (4) selon la revendication 9, dans lequel le dispositif de mélangeur (F ; F1, F2) comprend un bloc de permutation (MX), qui teste de façon cyclique toutes les combinaisons possibles des poids ($R_i$) de façon à trouver la combinaison optimale des poids ($R_i$) qui permet d'obtenir le meilleur signal combiné ($V_0$).

11.  Capteur (4) selon la revendication 10, dans lequel le dispositif de mélangeur (F ; F1, F2) comprend un bloc de reconnaissance QRS (R), qui analyse le signal combiné ($V_0$) et qui renvoie une valeur numérique (W) proportionnelle à la concordance du signal combiné ($V_0$) avec une onde QRS ; le meilleur signal combiné ($V_0$) étant le signal combiné ($V_0$) qui a la valeur numérique (W) la plus élevée renvoyée par le bloc de reconnaissance QRS (R).

12.  Capteur (4) selon la revendication 10 ou 11, comprenant :

un premier dispositif de mélangeur (F2), qui détermine la combinaison optimale des poids ($R_i$) qui permet d'obtenir le meilleur signal combiné ($V_0$) ; et
un deuxième dispositif de mélangeur (F1), qui utilise la combinaison optimale des poids ($R_i$) déterminée par le premier dispositif de mélangeur (F2) pour déterminer le signal combiné ($V_o$) .

13.  Véhicule (1), comprenant un compartiment passagers (2) équipé d'au moins un siège (3) dans lequel un capteur sans contact (4) selon l'une quelconque des revendications 1 à 12 est intégré pour détecter l'électrocardiogramme d'un utilisateur (5).

Fig. 1

Fig. 2

Fig. 3

Fig. 4

Fig. 5

Fig. 6

Fig. 7

EP 2 532 306 B1

Fig. 8

**EP 2 532 306 B1**

**REFERENCES CITED IN THE DESCRIPTION**

**Patent documents cited in the description**

- WO 02096694 A1
- JP 2011024902 A
- DE 10031822 A1
- US 2007255152 A1
- WO 2009070776 A1
- US 2008290937 A1
- US 3210678 A
- US 5650750 A
- US 4245649 A